# EUROPÄISCHE PATENTSCHRIFT

(11) **EP 1 240 102 B1**
(45) Veröffentlichungstag und Bekanntmachung des Hinweises auf die Patenterteilung: **27.10.2004**
(21) Anmeldenummer: 00967869.9
(22) Anmeldetag: 13.10.2000
(51) Int. Cl.: C01B 25/32, A61L 27/12

(54) **TRICALCIUMPHOSPHAT-HALTIGES HYDROXYLAPATIT-MATERIAL MIT MIKROPORÖSER STRUKTUR**
HYDROXYL APATITE MATERIAL CONTAINING TRICALCIUM PHOSPHATE, HAVING A MICROPOROUS STRUCTURE
HYDROXYAPATITE CONTENANT DU PHOSPHATE TRICALCIQUE, A STRUCTURE MICROPOREUSE

(30) Priorität: 18.10.1999 DE 19950113
(43) Veröffentlichungstag der Anmeldung: 18.09.2002
(73) Patentinhaber: Jordanova-Spassova, Margarita, 1404 Sofia (BG)
(72) Erfinder: Jordanova-Spassova, Margarita, 1404 Sofia (BG)
(74) Vertreter: Kleinschmidt, Michael, Dr.
(86) Internationale Anmeldenummer: PCT/EP2000/010117
(87) Internationale Veröffentlichungsnummer: WO 2001/028919

(56) Entgegenhaltungen:
- WO-A-98/54089
- DE-A- 3 709 897
- DE-C- 3 542 744
- US-A- 3 929 971
- DATABASE WPI Section Ch, Week 198445 Derwent Publications Ltd., London, GB; Class D21, AN 1984-278946 XP002158583 & JP 59 171545 A (YOSHIDA SEISAKUSHO KK), 28. September 1984 (1984-09-28)

## Beschreibung

Die Erfindung betrifft ein Tricalciumphosphat-haltiges Hydroxylapatit-Material und ein Verfahren zu seiner Herstellung.

Im Bereich der Knochenchirurgie besteht ein Bedarf an Knochenersatzstoffen und Implantaten, welche körperverträglich und gut zu bearbeiten sind. Um das Anwachsen im Körper zu erleichtern, sollte das Knochenimplantat eine Struktur aufweisen, die dem der Knochen möglichst ähnlich ist. Das Implantatmaterial sollte zudem als Träger für Wirkstoffe wie beispielsweise wachstumsfördernde oder -hemmende Substanzen geeignet sein.

Aus der US-PS 3 929 971 sind synthetische Materialen bekannt, welche ebenfalls aus dem Skelett von Korallen hergestellt werden sollen, wobei vorgeschlagen wird, das Verfahren so zu wählen dass die möglichst erhaltene Mikrostruktur der Korallen überwiegend entweder aus Tricalciumphosphat ("whitlockit") oder aus Hydroxylapatit bestehen sollen.

Als Knochenersatzmaterial hat sich ein Hydroxylapatit-Material grundsätzlich bewährt, welches aus dem Calciumcarbonat-Skelett von kalkinkrustierenden Algen gewonnen wird. Ein derartiges Material ist in der DE 37 09 897 C2 beschrieben. Um das natürlicherweise in den Algen vorhandene Magnesium zu maskieren und die Bildung von (β-Tricalciumphosphat während der Herstellung des Hydroxylapatit-Materials zu verhindern, wird dort vorzugsweise Fluorid zur Reaktionsmischung gegeben. Zur Herstellung von Formkörpern wird das durch hydrothermale Synthese erhaltene granuläre Hydroxylapatit-Material mit gelöschtem Kalk als Bindemittel in einen Formkörper eingerüttelt und danach nochmals einer hydrothermalen Behandlung unterzogen. Das so erhaltene Knochenimplantat besitzt eine hohe interkonnektive Porosität und eine hohe spezifische Oberfläche. In seinem Chemismus und kristallinen Aufbau ist es den Knochen wesentlich ähnlicher als andere Knochenersatzmaterialien.

Trotz der ausgezeichneten Eigenschaften des in der DE 37 09 897 C2 beschriebenen Hydroxylapatit-Materials ist es wünschenswert, ein Knochenersatzmaterial zur Verfügung zu haben, dessen Resorption im Organismus noch weiter verbessert ist und das zu einer noch schnelleren Knochenneubildung führt. Es hat sich nämlich herausgestellt, dass Hydroxylapatit-Material sich relativ zwar schon gute biologische Eigenschaften bei der Versorgung im Körper hat, dass aber eine lange biologische Abbauzeit in Kauf genommen werden muss. Diese Tatsache insbesondere für Hydroyxlapatit-Materialien mit Kristallgrößen in der Größenordnung von einigen Mikrometern mit einem Ca/P Verhältnis von 1,67 ist aus der WO 97/17285 A1, aber auch aus der WO94/02412 A1 bekannt. Aber auch für die aus der DE 37 09 897 C2 bekannten Hydroyxlapatit-Materialien, bei denen die interkonnektive Porenstruktur der kalkinkrustierenden Algen erhalten bleibt, ist die biologische Abbauzeit größer als z.B. bei Tricalciumphosphat. Andererseits ist die biologische Abbauzeit von Tricalciumphosphat wesentlich geringer.

Wünschenswert ist eine biologische Abbauzeit, die in etwa mit der Zeit synchronisiert ist, mit der der Knochen an Stelle des abgebauten Knochenersatzmaterials nachwächst. Dies führt zu der Forderung an das Knochenersatzmaterial, dass bei der Herstellung die Abbauzeit zumindest in bestimmten Grenzen eingestellt werden kann und so eine beschleunigte Knochenbildung gefördert wird, ohne dass das Material zu schnell abgebaut würde und so ein z.B. mit Bindegewebe gefüllter Zwischenraum entsteht.

Aufgabe der Erfindung ist es, ein Knochenersatzmaterial anzugeben, welches eine hohe interkonnektive Porenstruktur aufweist, sehr gut körperverträglich ist sowie eine verbesserte und kontrollierbare Resorption im Organismus aufweist und so zu einer beschleunigten Knochenneubildung führt. Das Knochenersatzmaterial sollte außerdem als Träger für Wirkstoffe wie beispielsweise wachstumsfördernde oder - hemmende Substanzen geeignet sein.

Die Lösung der Aufgabe gelingt mit dem Tricalciumphosphat-haltigen Hydroxylapatit-Material mit mikroporöser Struktur nach Anspruch 8. Die Erfindung betrifft weiterhin ein Verfahren zu Herstellung dieses Materials nach Ansprüchen 1 und 2. Bevorzugte Ausführungsformen und Verfahrensweisen sind in den Unteransprüchen dargelegt.

Das Tricalciumphosphat-haltige Hydroxylapatit-Material nach der vorliegenden Erfindung zeichnet sich durch einen Tricalciumphosphat-Gehalt aus, der wesentlich höher ist als derjenige eines Hydroxylapatit-Material, welches auf herkömmliche Weise aus kalkinkrustierenden Algen gewonnen wurde.

Der Gehalt an β-Tricalciumphosphat (whitlockit) in Hydroxylapatit-Material, welches aus kalkinkrustierenden Algen gewonnen wurde, hängt von der Menge an Magnesiumionen ab, welche bei der Herstellung des Hydroxylapatit-Materials vorhanden sind. Wie bereits eingangs erwähnt, besitzen natürlich vorkommende Algen von Natur aus einen bestimmten Gehalt an Magnesium. Dieser natürliche Gehalt an Magnesium in Algen beträgt normalerweise ca. 2,5%, in Ausnahmefällen auch bis zu etwa 6 Gew.-%. Dies führt zu einem Gehalt von bis zu 10 Gew.-%, in Ausnahmefällen auch bis zu etwa 20 Gew.-%, β-Tricalciumphosphat in einem nach DE 37 09 897 C2 aus diesen Algen hergestellten Hydroxylapatit-Material. Es ist dabei zu bemerken, dass in den Algen das Magnesium in einer zumindest teilweise in die Struktur eingebundenen Form vorliegt, nämlich vorwiegend in Form von MgCO₃, MgO und (Ca, Mg)CO₃, was offenbar dazu führt, dass mit diesem Magnesium nicht nur β-Tricalciumphosphat erzeugt wird, sondern auch andere, störende Phasen mit Magnesiumanteil erzeugt werden. In der DE 37 09 897 C2 war es jedoch als nachteilig angesehen worden, dass bei der Herstellung des Hydroxylapatit-Materials Tricalciumphosphat gebildet wird. Aus diesem Grund wird dort in einer bevorzugten Herstellungsvariante das natürlicherweise in dem Algen-Ausgangsmaterial vorhandene Magnesium durch gezielte Zugabe von Fluoridionen maskiert, um die Bildung des Tricalciumphosphats zu unterdrücken.

Der Erfindung liegt nun die Erkenntnis zugrunde, dass ein Hydroxylapatit-Material mit einem Tricalciumphosphat-Gehalt, welcher höher ist als derjenige Gehalt, der sich bei Verwendung einer bestimmten Algenart als Ausgangsmaterial automatisch einstellen würde, im Hinblick auf die Resorption im Organismus und die Förderung der Knochenneubildung gegenüber dem herkömmlichen Hydroxylapatit-Material verbessert ist, ohne dass sich die anderen vorteilhaften Eigenschaften verschlechtern. Dieser erhöhte Tricalciumphosphat-Gehalt wird durch gezielten Zusatz von Magnesiumionen während der Herstellung des Materials erreicht.

Die Erfindung betrifft also ein Tricalciumphosphat-haltiges Hydroxylapatit-Material, welches erhältlich ist durch Umsetzen eines von organischen Verbindungen befreiten Algen-Hartgewebes in einer alkalischen wässrigen Phosphat-Lösung unter Zusatz von Magnesiumionen bei erhöhter Temperatur. Durch den Zusatz der Magnesiumionen wird der Gehalt an Magnesium in der Reaktionsmischung also über das Maß hinaus gesteigert, welches dem natürlichen Magnesiumgehalt der entsprechenden Algenart, welche als Ausgangsmaterial der Synthese eingesetzt wird, entspricht. Entsprechend steigt damit auch der Tricalciumphosphat-Gehalt im so hergestellten Hydroxylapatit-Material.

Erfindungsgemäß ist es möglich, durch gezielte Zugabe von Magnesiumionen zur Reaktionsmischung den Tricalciumphosphat-Gehalt im Endprodukt auf einen vorgegebenen gewünschten Wert einzustellen. Bevorzugt liegt der Tricalciumphosphat-Gehalt im erfindungsgemäßen Hydroxylapatit-Material zwischen 50 und 90 Gew.-%.

Um einen entsprechenden Tricalciumphosphat-Gehalt in Hydroxylapatit-Material zu erreichen, ist ein Zusatz von 0,1 bis ca. 15 Gew.-% Magnesiumionen in Form eines Magnesiumsalzes zu dem Gemisch der festen Ausgangsmaterialien (Algen-Hartgewebe und Phosphatsalz) denkbar. Die Zugabe der Magnesiumionen darf 15 Gew.-% nicht überschreiten, da sich sonst ein zu große Menge anderer unerwünschter magnesiumhaltiger Phasen bildet, wie z. β-Dittmarit (MgNH₄PO₄•6H₂O). Für das Tricalciumphosphat-haltige Hydroxylapatit-Material mit mikroporöser Struktur entsprechend dem Algenhartgewebe gemäß der vorliegenden Erfindung sollten die Mg²⁺-Gehalte in der Mischung der festen Ausgangsmaterialien zwischen 0,75 und 6 Gew.-% liegen.

Wie bereits erwähnt, vereinigt das erfindungsgemäße Tricalciumphosphat-haltige Hydroxylapatit-Material alle Vorteile in sich, die bereits aus dem in der DE 37 09 897 C2 beschriebenen Material bekannt sind. Das erfindungsgemäße Hydroxylapatit-Material behält die mikroporöse Struktur des Algen-Hartgewebes, welches als Ausgangsmaterial eingesetzt wurde, bei und weist somit eine sehr hohe interkonnektive Porosität auf. Wegen der großen Porosität ist das erfindungsgemäße Hydroxylapatit-Material auch als Filtermaterial ausgezeichnet einsetzbar. Die spezifische Oberfläche des Materials ist sehr hoch. Das Material ist sehr gut körperverträglich und in seinem Chemismus knochenähnlich, so dass es sich ausgezeichnet als Knochenersatzmaterial eignet. Durch den erhöhten Tricalciumphosphat-Gehalt wird es gegenüber dem aus der DE 37 09 897 C2 bekannten Material jedoch noch besser vom Organismus resorbiert und beschleunigt die Knochenneubildung. Über die Konzentration der Magnesiumionen sind zudem der Tricalciumphosphat-Gehalt und damit die resorptiven Eigenschaften gezielt einstellbar. Mit zunehmendem Tricalciumphosphat-Gehalt wird die Resorption im Körper schneller. Umgekehrt verlangsamt ein höherer Hydroxylapatit-Gehalt die Resorption. Das erfindungsgemäße Hydroxylapatit-Material eignet sich außerdem ausgezeichnet als Trägermaterial für Wirkstoffe wie beispielsweise wachstumsfördernde oder -hemmende Substanzen. Spezielle Beispiele sind Antibiotika, Chemotherapeutika, tumorhemmende Verbindungen und knocheninduktive Substanzen. Als knocheninduktive Substanzen können beispielsweise knochenmorphogene Proteine (bone morphogenic proteins, BMP) genannt werden.

In einer bevorzugten erfindungsgemäßen Ausführungsform weist das Tricalciumphosphat-haltige Hydroxylapatit-Material einen Gehalt an wenigstens einem Wirkstoff auf. Dieser kann entweder in das erfindungsgemäße Hydroxylapatit-Material oder in das hieraus hergestellte Knochenersatzmaterial eingearbeitet sein, oder er ist als Beschichtung auf einem Knochenersatzmaterial vorhanden, welches unter Verwendung des erfindungsgemäßen Tricalciumphosphat-haltige Hydroxylapatit-Materials hergestellt wurde. Zweckmäßig wird der wenigstens eine Wirkstoff in einer klinisch aktiven Menge auf das Hydroxylapatit-Material oder das Knochenersatzmaterial aufgebracht oder in dieses eingemischt.

Als Algen-Hartgewebe, welches als Ausgangsmaterial für das erfindungsgemäße Hydroxylapatit-Material verwendet wird, kann grundsätzlich jedes Algen-Hartgewebe verwendet werden, das bisher bereits zur Herstellung von Hydroyxlapatit-Materialien verwendet wurde. Besonders geeignet ist Algen-Hartgewebe, welches aus kalkinkrustierenden Meeresalgen gewonnen wurde. Beispielhaft für diese Meeresalgen können die Spezies Amphiroa Ephedra, Corallinacea oder Codiacea genannt werden. Diese Algen werden zweckmäßig mazeriert, also beispielsweise ca. einen Tag lang bei 700°C pyrolisiert und anschließend granuliert. Das sc erhaltene Algen-Hartgewebe besteht überwiegend aus Calciumcarbonat (ca. 95 Gew.-%), die restlichen etwa fünf Gew.-% sind hauptsächlich Calciumoxid und Magnesiumoxid.

Im erfindungsgemäßen Verfahren zur Herstellung eines Tricalciumphosphat-haltigen Hydroxylapatit-Materials wird dieses von organischen Verbindungen befreite Algen-Hartgewebe in einer alkalischen wässrigen Phosphat-Lösung unter Zusatz eines Magnesiumsalzes bei erhöhter Temperatur umgesetzt. Das Verhältnis von Algenhartgewebe und Phosphatsalz wird dabei auf an sich bekannte Weise so gewählt, dass das Verhältnis von Calcium zu Phosphor dem Verhältnis im Hydroxylapatit entspricht. Das Verhältnis von Calcium zu Phosphor im Hydroxylapatit liegt bei etwa 1,67. Verwendet man als Phosphatsalz beispielsweise Diammoniumhydrogenphosphat, liegt das Gewichtsverhältnis von Algen-Hartgewebe zu Phosphatsalz üblicherweise im Bereich von 1 : 0,8 bis 1 : 1,5, vorzugsweise bei 1 : 0,95 bis 1 : 1. Das Gewichtsverhältnis schwankt etwas in Abhängigkeit vom Calciumgehalt des verwendeten Algen-Hartgewebes.

Bezüglich des Gehaltes an Magnesiumionen in der Reaktionsmischung kann auf das Vorstehende verwiesen werden. Zweckmäßig wird das Magnesiumsalz in einer solchen Menge zugesetzt, dass der Gehalt von Tricalciumphosphat im Endprcdukt zwischen 50 und 90 Gew.-% liegt. Als bevorzugte Quelle der Magnesiumionen wird Magnesiumnitrat eingesetzt. Es können jedoch grundsätzlich auch andere wasserlösliche Magnesiumsalze verwendet werden. Als bevorzugte Phosphatverbindung dient, wie bereits erwähnt, Diammoniumhydrogenphosphat. Das erfindungsgemäße Verfahren ist jedoch nicht auf dieses Salz beschränkt.

Die Umsetzung der oben genannten Reaktionskomponenten erfolgt im Alkalischen bei erhöhter Temperatur. Ein bevorzugter pH-Bereich liegt zwischen 8 und 11. Besonders zweckmäßig erfolgt die Umsetzung bei einem pH-Wert zwischen 9 und 9,5. Zur Einstellung des pH-Wertes können grundsätzlich alle wasserlöslichen Basen verwendet werden. Besonders bevorzugt wird Ammoniak eingesetzt.

Ein geeigneter Temperaturbereich für die Durchführung des erfincungsgemäßen Verfahrens liegt zwischen 150 und 250°C, insbesondere zwischen 200 und 250°C. Ein besonders bevorzugter Temperaturbereich liegt zwischen 230 und 250°C. Bei dieser Temperatur wird die Umsetzung üblicherweise wenigstens 24 Stunden dauern.

Das erfindungsgemäße Verfahren wird zweckmäßig bei erhöhtem Druck durchgeführt. Es hat sich als besonders geeignet erwiesen, die Umsetzung in einem Autoklaven durchzuführen. Vorzugsweise wird ein mit einer inerten Beschichtung versehener Autoklav verwendet. Geeignet ist insbesondere ein mit Polytetrafluorethylen ausgekleideter Autoklav.

Besonders bevorzugt ist es, die hydrothermale Umsetzung zum erfindungsgemäßen Tricalciumphosphat-haltigen Hydroxylapatit-Material bei dem Sättigungsdampfdruck der Reaktionslösung durchzuführen. Damit sich während der Umsetzung der Sättigungsdampfdruck einstellen kann, wird der Autoklav zweckmäßig maximal zu zwei Dritteln gefüllt.

Die Erfindung soll nachfolgend anhand eines Beispiels weiter beschrieben werden.

### Beispiel 1

### Herstellung des erfindungsgemäßen Tricalciumphosphat-haltigen Hydroxylapatit-Material

(a) Ein durch Mazerieren von Algen der Spezies Corallina officinalis gewonnenes Algen-Hartmaterial wird in Form eines Granulats mit einer Korngröße von über 0,5 mm in einen mit Polytetra-Fluorethylen ausgekleideten Autoklaven gefüllt. Dem Autoklaven wird weiterhin Phosphat zugesetzt, und zwar in einer solchen Menge, dass das Verhältnis von Calcium zu Phosphor im Hydroxylapatit im Bereich von 1,67 liegt. Als Phosphatsalz wird (NH₄)₂HPO₄ verwendet. Das Gewichtsverhältnis von Algen-Hartgewebe zum Phosphatsalz beträgt 1 : 0,95. Das Phosphatsalz wird in Form einer wässrigen Lösung zugegeben, wobei das Gewichtsverhältnis von Phosphatsalz zu Wasser etwa 1 : 4 beträgt. Weiterhin wird dem Autoklaven Magnesium in Form von Mg (NO₃)₂•6H₂O zugesetzt. Die Menge beträgt 2,5 Gew.-% Magnesium, bezogen auf den Feststoffgehalt im Autoklaven.
(b) Durch Einleiten von Ammoniak in die Reaktionsmischung wird der pH-Wert auf einen Wert zwischen 9 und 9,5 eingestellt. Es ist darauf zu achten, dass der Autoklav nicht mehr als bis zu zwei Dritteln gefüllt ist.
(c) Die hydrothermale Umsetzung wird bei einer Temperatur zwischen 230 und 250°C mindestens 24 Stunden lang durchgeführt. Die Umsetzung erfolgt beim Sättigungsdampfdruck der Lösung.
(d) Nach Beendigung der hydrothermalen Reaktion wird der Autoklav abgekühlt. Anschließend wird der pH-Wert kontrclliert. Liegt der pH-Wert nicht mehr in dem Bereich, der zu Beginn der Reaktion eingestellt wurde, wird die Reaktionsmischung verworfen. Liegt der pH-Wert im gewünschten Bereich, wird die wässrige Phase abgetrennt und verworfen. Das isolierte Tricalciumphosphat-haltige Hydroxylapatit-Material wird mehrfach mit warmem destillierten Wasser gewaschen und dabei gegebenenfalls kurz aufgekocht. Nach dem Waschen wird das erhaltene Tricalciumphosphat-haltige Hydroxylapatit-Material bei einer Temperatur von bis zu 200°C in einem Ofen getrocknet.

### Beispiel 2

### Herstellung des erfindungsgemäßenTricalciumphosphat-haltigen Hydroxylapatit-Material

Die Verfahrensschritte (a), (b) und (d) entsprechen dem Beispiel 1, jedoch wird die Synthese in einem Temperaturbereich von 200°C bis 240°C durchgeführt. Die Temperatur in dieser Ausführung variiert, wobei die Synthese zunächst für zwei Stunden bei einer Temperatur von 200°C abläuft. Dann wird die Temperatur für die nächsten zwei Stunden auf 240°C erhöht, darauf folgend wieder auf 200°C eingestellt. Dieser Zyklus wird wiederum für insgesamt 24 Stunden beibehalten. Die Veränderung der Temperatur in den angegebenen grenzen führt zu einer hohen, aber gleichzeitig sehr feinen Kristallunität des Produktes.

Die beiden Phasen (Hydroxylapatit und Tricalciumphosphat) bilden sehr unterschiedliche Kristallite, auch mit sehr unterschiedlicher Größe. Die Tricaliciumphase bildet viel größere Kristallite als die Apatitphase. Wenn diese Kristallisationsprozesse frei laufen gelassen würden, d.h. bei einer konstanten Temperatur, könnten eben diese Unterschiede in der Bildung der beiden Phasen zu einer Zerstörung der wertvollen Mikrostruktur des Algenskeletts führen. Die Trennwände im Kanalsystem des Skeletts sind nämlich aus unterschiedlichen Konglomeraten aufgebaut, was zu einer Destabilisierung der Struktur bei unzureichend ausgewählten Prozessparametern führt. Die Umsetzung erfolgt wiederum beim Sättigungsdampfdruck der Lösung.

Die Veränderung der Sintertemperatur verändert während des gesamten Ablaufs die thermodynamischen Bedingungen im Autoklaven, so dass die Kristallisationsprozesse zwar ablaufen, aber das Wachstum der Kristallite gebremst wird und diese am Ende klein bleiben. Dies ist ein Verfahren zur Erhaltung der Stabilität des Algenskeletts.

Als Ergebnis dieser Ausführung kann folgendes festgestellt werden. Bei einem herkömmlichen Ablauf der Synthese hat die Tricalciumphosphatphase eine Kristallitgröße von 100 bis 150 nm und die Apatitphase eine Kristallitgröße von ca. 50 nm. Nach dem hier angegebenen Beispiel 2 kann für die Apatitphase eine Kristallitgröße von 30 nm und für die Tricalciumphosphatphase von 40 bis 45 nm erreicht werden.

Als alternative Ausführungsform kann die Temperatur der hydrothermalen Synthese auch alle vier Stunden zwischen 200°C und 240°C variiert werden.

Die höchsten Konzentrationen von Tricalciumphosphat in dem Produkt entsprechend der vorliegenden Erfindung beträgt ca. 90%, wobei der Rest hauptsächlich Hydroxylapatit ist. Die Struktur ist stabil und die einzelnen Kerne des Granulats sind genügend hart. Der maximale Mg-Zusatz beträgt 5,5 bis 6,0 Gew.-%, berechnet auf die Menge der gebrannten Algen (Calcit-Hartgewebe). Bei einer gewünschten Konzentration von 50% Tricalciumphosphat (bei ca. 50% Hydroxylapatit) beträgt die Zugabe ca. 0,75 Gew.-%, bei einer gewünschten Konzentration von 60% ca. 3,0 Gew.-%. Es gibt also einen Sprung in Bezug auf die Zugabe von Mg, die nötig ist, um eine Konzentration von Tricalciumphosphat von deutlich über 50% zu erreichen. Eine solche Konzentration lässt sich mit dem beschriebenen, besonders geeigneten Verfahrensweise der Temperaturvariation erzielen. Bei sehr hohen Anteilen von Tricalciumphosphat in der Hydroxylapatitmasse, zu deren Erzeugung die Obergrenze der erwähnten Magnesiummenge verwendet wird, können kristalline Formen von Ablagerungen mit Magnesiumanteil unter anderem von β-Dittmarit (MgNH₄PO₄•6H₂O) vermieden werden, indem das Produkt mit schwacher Essigsäure und Aqua Bidest. mehrmals gewaschen wird.

Damit wird der überschüssigen, aber zur Reaktion notwendigen Menge Magnesium die Funktion eines Katalysators gegeben, die der natürliche Anteil an Magnesium in den gebrannten Algen in Form von in das Kristallnetz eingebundenem MgCO₃, MgO und (Ca, Mg)CO₃ nicht hat.

## Patentansprüche

1. Verfahren zur Herstellung eines Tricalciumphosphat-haltiges Hydroxylapatit-Material durch Umsetzen eines von organischen Verbindungen befreiten Algen-Hartgewebes in einer alkalischen wässrigen Phosphat-Lösung unter Zusatz von Mg²⁺-Ionen bei erhöhter Temperatur unter Beibehaltung der mikroporösen Struktur des Algen-Hartgewebe-Ausgangsmaterials und einem Tricalciumphosphat-Gehalt von 50 bis 90 Gew.-%, vorzugsweise von 70% - 90%, im Endprodukt, wobei ein von organischen Verbindungen befreites Algen-Hartgewebe, insbesondere aus kalkinkrustierenden Meeresalgen und insbesondere aus solchen der Spezies Amphiroa Ephedra, Corallinacea oder Codiacea gewonnenes Algen-Hartgewebe in einer alkalischen wässrigen Phosphat-Lösung unter Zusatz eines wasserlöslichen Magnesium-Salzes bei erhöhter Temperatur umgesetzt wird, wobei das Magnesium - Salz in solcher Menge eingesetzt wird, dass im Endprodukt ein Gehalt von 50 bis 90 Gew%, vorzugsweise 70% bis 90%, Tricalciumphosphat erhalten wird, und wobei die Reaktionstemperatur zwischen 230°C und 250°C liegt.

2. Verfahren zur Herstellung eines Tricalciumphosphat-haltiges Hydroxylapatit-Material durch Umsetzen eines von organischen Verbindungen befreiten Algen-Hartgewebes in einer alkalischen wässrigen Phosphat-Lösung unter Zusatz von Mg²⁺-Ionen bei erhöhter Temperatur unter Beibehaltung der mikroporösen Struktur des Algen-Hartgewebe-Ausgangsmaterials und einem Tricalciumphosphat-Gehalt von 50 bis 90 Gew.-%, vorzugsweise von 70% - 90%, im Endprodukt, wobei ein von organischen Verbindungen befreites Algen-Hartgewebe, insbesondere aus kalkinkrustierenden Meeresalgen und insbesondere aus solchen der Spezies Amphiroa Ephedra, Corallinacea oder Codiacea gewonnenes Algen-Hartgewebe in einer alkalischen wässrigen Phosphat-Lösung unter Zusatz eines wasserlöslichen Magnesium-Salzes bei erhöhter Temperatur umgesetzt wird, wobei das Magnesium-Salz in solcher Menge eingesetzt wird, dass im Endprodukt ein Gehalt von 50 bis 90 Gew.-%, vorzugsweise von 70% bis 90%, Tricalciumphosphat erhalten wird, **dadurch gekennzeichnet, dass** die Reaktionstemperatur zwischen 200 und 240°C variiert wird, wobei die Reaktionstemperatur jeweils in Zyklen von ca. 200° und ca. 240°C gehalten wird.

3. Verfahren nach Anspruch 2, **dadurch gekennzeichnet, dass** die genannten Zyklen jeweils zwei Stunden betragen.

4. Verfahren nach einem der vorstehenden Ansprüche, **dadurch gekennzeichnet, dass** als wasserlösliches Magnesium-Salz Magnesiumnitrat eingesetzt wird.

5. Verfahren nach einem der Ansprüche 1 bis 4, **dadurch gekennzeichnet, dass** als Phosphat (NH₄)₂HPO₄ eingesetzt wird.

6. Verfahren nach einem der Ansprüche 1 bis 5, **dadurch gekennzeichnet, dass** die Umsetzung bei einem pH-Wert zwischen 8 und 11 und insbesondere zwischen 9 und 9,5 durchgeführt wird, wobei der pH-Wert mit NH₃ eingestellt wird.

7. Verfahren nach einem der Ansprüche 1 bis 6, **dadurch gekennzeichnet, dass** die Umsetzung bei erhöhtem Druck durchgeführt wird und dass die Umsetzung in einem Autoklaven und insbesondere in einem mit Polytetrafluorethylen ausgekleideten Autoklaven erfolgt.

8. Tricalciumphosphat-haltiges Hydroxylapatit-Material, erhältlich durch Umsetzen eines von organischen Verbindungen befreiten Algen-Hartgewebes nach einem der Ansprüche 1 bis 7, **dadurch gekennzeichnet, dass**
- die mikroporöse Struktur des Algen-Hartgewebe-Ausgangsmaterials im Endprodukt erhalten geblieben ist und
- das Material einen Tricalciumphosphat-Gehalt von 50 bis 90 Gew.-%, vorzugsweise von 70%-90%, aufweist.

9. Tricalciumphosphat-haltiges Hydroxylapatit-Material nach Anspruch 8, **gekennzeichnet durch**
einen Gehalt an wenigstens einem Wirkstoff, insbesondere einer wachstumsfördernden oder wachstumshemmenden Verbindung, einem Antibiotikum, Chemotherapeutikum, einer tumorhemmenden oder knocheninduktiven Verbindung, insbesondere wenigstens einem Knochen-morphogenen Protein.

## Revendications

1. Procédé pour fabriquer un matériau d'hydroxylapatite contenant du phosphate tricalcique en transformant un tissu solide d'algues libéré de composés organiques dans une solution de phosphate alcaline et aqueuse en ajoutant des ions Mg²⁺ à température élevée et en conservant la structure microporeuse du matériau de départ du tissu solide d'algues en maintenant un taux de phosphate tricalcique de 50 à 90 % en poids, de préférence, 70% - 90 % en poids dans le produit final; un tissu solide d'algues libéré des composés organiques, notamment composé d'algues de mer incrustant du calcaire et notamment de celles du tissu solide d'algues de l'espèce d'algues Amphiroa Ephedra, Corallinacea ou Codiacea en une solution de phosphate alcaline et aqueuse en ajoutant un sel de magnésium soluble dans l'eau à température élevée ; le sel de magnésium étant introduit en une telle quantité pour obtenir dans le produit final un taux de 50 à 90 % en poids, de préférence 70% à 90% de phosphate tricalcique ; la température de réaction étant située entre 230°C et 250°C.

2. Procédé pour fabriquer un matériau d'hydroxylapatite contenant du phosphate tricalcique en transformant un tissu solide d'algues libéré des composés organiques en une solution de phosphate alcaline et aqueuse en ajoutant des ions Mg²⁺ à température élevée et en conservant la structure microporeuse et un taux de phosphate tricalcique de 50 à 90 % en poids, de préférence de 70% - 90%, de préférence, 70% - 90 % dans le produit final; un tissu solide d'algues libéré des composés organiques, notamment composé d'algues de mer incrustant du calcaire et notamment de celles du tissu solide d'algues de l'espèce d'algues Amphiroa Ephedra, Corallinacea ou Codiacea en une solution de phosphate alcaline et aqueuse en ajoutant un sel de magnésium soluble dans l'eau à température élevée ; le sel de magnésium étant introduit en une telle quantité avoir d'avoir dans le produit final un taux de 50 à 90 % en poids, de préférence 70% à 90% de phosphate tricalcique, **caractérisé en ce que** la température de réaction varie entre 200 et 240°C ; la température de réaction étant maintenue à chaque fois en cycles d'environ 200 et 240°C.

3. Procédé selon la revendication 2, **caractérisé en ce que** les cycles mentionnés s'élèvent à chaque fois à deux heures.

4. Procédé selon une des revendications précédentes, **caractérisé en ce qu'**on utilise du nitrate de magnésium comme sel de magnésium soluble dans l'eau.

5. Procédé selon une des revendications 1 à 4, **caractérisé en ce qu'**on utilise du phosphate (NH₄)₂HPO₄.

6. Procédé selon une des revendications 1 à 5, **caractérisé en ce que** la transformation est réalisée à un pH situé entre 8 et 11, notamment entre 9 et 9,5 ; la valeur du pH étant réglée avec du NH₃.

7. Procédé selon une des revendications 1 à 6, **caractérisé en ce que** la transformation est réalisée à une pression élevée et **en ce que** la transformation se déroule dans des autoclaves et notamment dans des autoclaves habillés avec du polytetrafluoréthylène.

8. Matériau d'hydroxylapatite contenant du phosphate tricalcique obtenu par la transformation d'un tissu solide d'algues libéré des composés organiques selon une des revendications 1 à 7, **caractérisé en ce que** :
- la structure microporeuse du matériau de sortie du tissu solide d'algues a été maintenue et
- le matériau présente un taux de phosphate tricalcique de 50 à 90 % en poids, de préférence 70%-90%.

9. Matériau d'hydroxylapatite contenant du phosphate tricalcique selon la revendication 8, **caractérisé par** un taux d'au moins une substance active, notamment d'un composé favorisant la croissance ou d'un composé freinant la croissance, un antibiotique, une substance chimiothérapeutique , un composé freinant la croissance d'une tumeur ou formant l'os, notamment au moins, une protéine morphogène de l'os.

## Claims

1. A process for producing a hydroxylapatite material containing tricalcium phosphate by converting a hard algae tissue free of organic compounds in an alkaline aqueous phosphate solution with addition of Mg²⁺ ions at increased temperature, wherein the microporous structure of the hard algae tissue starting materials remains intact and has a tricalcium phosphate content of 50 to 90 % by weight, preferably 70% to 90% by weight in the end product, wherein hard algae tissue, in particular obtained from lime-encrusting sea algae, in particular obtained from such species as Amphiroa Ephedra, Corallinacea or Codiacea, free from organic compounds, is converted in an alkaline aqueous phosphate solution with addition of a water soluble Magnesium salt at increased temperature, wherein the magnesium salt is used in such an amount that the end product has a tricalcium phosphate content of 50 to 90 % by weight, preferably 70% to 90% by weight and whereby the reaction temperature is between 230°C and 250°C.

2. A process for producing a hydroxylapatite material containing tricalcium phosphate by converting a hard algae tissue free of organic compounds in an alkaline aqueous phosphate solution with addition of Mg²⁺ ions at increased temperature, wherein the microporous structure of the hard algae tissue starting materials remains intact and has a tricalcium phosphate content of 50 to 90 % by weight, preferably 70% to 90% by weight in the end product, wherein hard algae tissue, in particular obtained from lime-encrusting sea algae, in particular obtained from such species as Amphiroa Ephedra, Corallinacea or Codiacea, free from organic compounds, is converted in an alkaline aqueous phosphate solution with addition of a water soluble Magnesium salt at increased temperature, wherein the magnesium salt is used in such an amount that the end product has a tricalcium phosphate content of 50 to 90 % by weight, preferably 70% to 90% by weight, **characterized in that** the reaction temperature is varied between 200°C and 240°C, whereby the reaction temperature is maintained in cycles at 200°C and 240°C, respectively.

3. The process as claimed in Claim 2, **characterised in that** the abovementioned cycles are two hours in each case.

4. The process as claimed in any of the above-mentioned claims, **characterised in that** magnesium nitrate is used as water-soluble magnesium salt.

5. The process as claimed in any one of Claims 1 to 4, **characterised in that** (NH₄)₂HPO₄ is used as phosphate.

6. The process as claimed in any one of Claims 1 to 5, **characterised in that** conversion is carried out at a pH value between 8 and 11 and in particular between 9 and 9.5.

7. The process as claimed in any one of Claims 1 to 6, **characterised in that** conversion is carried out at increased pressure and that the conversion takes place in an autoclave and in particular in an autoclave coated with polytetrafluorethylene.

8. A hydroxylapatite material containing tricalcium phosphate, obtained by converting a hard algae tissue free of organic compounds according to any of claims 1 to 7, **characterised in that**
- the microporous structure of the hard algae tissue starting materials remains intact in the end product, and
- the material has a tricalcium phosphate content of 50 to 90 % by weight, preferably 70% to 90% by weight.

9. The hydroxylapatite material containing tricalcium phosphate as claimed Claims 8, **characterised by** a content of at least one active ingredient, in particular a growth-promoting or growth-inhibiting compound, an antibiotic, chemotherapeutic, a tumor-inhibiting or bone-inductive compound, in particular at least one bone-morphogenic protein.
